(19) Europäisches Patentamt / European Patent Office / Office européen des brevets

(11) **EP 4 368 606 B1**

(12) **EUROPEAN PATENT SPECIFICATION**

(45) Date of publication and mention
of the grant of the patent:
**18.06.2025  Bulletin 2025/25**

(21) Application number: **23207491.4**

(22) Date of filing: **02.11.2023**

(51) International Patent Classification (IPC):
$C07C\ 51/09^{(2006.01)}$       $C07C\ 51/44^{(2006.01)}$
$C07C\ 53/08^{(2006.01)}$       $C07C\ 67/54^{(2006.01)}$
$C07C\ 69/80^{(2006.01)}$       $C08L\ 1/12^{(2006.01)}$

(52) Cooperative Patent Classification (CPC):
(C-Sets available)
**C07C 51/09; C07C 51/44; C07C 67/54; C08L 1/12**
(Cont.)

(54) **PROCESS FOR RECOVERING ACETIC ACID AND OTHER VALUABLE MATERIALS FROM CELLULOSE ACETATE WASTE, BY MEANS OF PYROLYSIS**

VERFAHREN ZUR GEWINNUNG VON ESSIGSÄURE UND ANDEREN WERTSTOFFEN AUS CELLULOSEACETATABFÄLLEN MITTELS PYROLYSE

PROCÉDÉ DE RÉCUPÉRATION D'ACIDE ACÉTIQUE ET D'AUTRES MATIÈRES VALORISABLES À PARTIR DE DÉCHETS D'ACÉTATE DE CELLULOSE, PAR PYROLYSE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **11.11.2022  IT 202200023352**

(43) Date of publication of application:
**15.05.2024  Bulletin 2024/20**

(73) Proprietors:
• **Acetek Momentum Co., Ltd.**
**Zaozhuang City Shandong (CN)**
• **Jinan Acetate Chemical Co., Ltd.**
**Jinan City Shandong (CN)**

(72) Inventors:
• **De Filippis, Paolo**
**Rome (IT)**
• **De Caprariis, Benedetta**
**Rome (IT)**

(74) Representative: **Sarpi, Maurizio et al**
**Studio Ferrario S.r.l.**
**Via Collina, 36**
**00187 Roma (IT)**

(56) References cited:
CN-A- 107 673 962      CN-A- 112 047 827

(52) Cooperative Patent Classification (CPC): (Cont.)

C-Sets
**C07C 51/09, C07C 53/08;
C07C 51/44, C07C 53/08;
C07C 67/54, C07C 69/80**

# EP 4 368 606 B1

**Description**

The present invention relates to an innovative process for recovering acetic acid useful for the production of acetic anhydride and other valuable materials from cellulose acetate waste and scrap, by means of pyrolysis.

CN112047827A discloses a method for obtaining acetic acid from waste cellulose acetate slurry, which comprises an acid degradation treatment carried out in the presence of sulfuric acid at 90-100°C. Then, acetic acid is extracted with isopropyl acetate and separated by distillation.

The Applicants have experimentally demonstrated that pyrolysis advantageously allows the selective recovery of acetic acid and diethyl phthalate which, at a temperature of 370 °C, are 30% by weight of the total feed for acetic acid, and about 20% for diethyl phthalate. It further highlights that, since the two compounds have a very different boiling temperature, the separation can be carried out very easily, for example in a distillation column.

## SCOPE OF THE INVENTION

The recovery of cellulose acetate waste and scrap takes a primary economic value, especially in the production of eyeglasses.

It is known that eyeglasses have become a fashion accessory in recent decades which, in addition to responding to the need to accommodate a pair of corrective sun lenses, have taken on an aesthetic value. For this reason, numerous fashion brands are present on the distribution market worldwide, reaffirming the brand concept and the perceived value of a pair of eyeglasses.

Plastic eyeglasses are better suited to the role of eyeglasses as an expression of fashion as, in addition to the model, plastic creates games of colors and structures through the three-dimensionality thereof.

When mention is made of plastic, reference is mostly made to eyeglasses milled from a sheet of cellulose acetate, a polymer of natural origin which is synthesized from cotton or wood pulp with the addition of acetic acid. The production of eyeglasses from sheets includes cutting a rectangle (170x70mm) from a sheet of different sizes (600x1400m; 170x1400mm; 170x700mm). The thickness of the sheet varies from 5 to 8 m.

The weight of the eyeglasses can be about 20 g, while the rectangle can weigh from 92 g to 122 g, with waste therefore ranging from 78% to 84%. The eyeglasses production process, although virtuous from the "aesthetic" point of view, is flawed from the point of view of "efficiency," as it registers significant scraps in sheets which go partly to landfill and are partly used as a by-product (in the form of granules obtained from the extrusion of the waste) for items without performance expectations.

To this, the waste of the acetate sheet production must be added, according to the extrusion or solvent manufacturing process. Part of this waste is reused in the sheet manufacturing process itself and part is destined for the production of semi-finished products for products without performance expectations.

The sheet production centers are in Italy for about 3,000 tons/year and China for about 16,000 tons/year and the eyeglasses production centers are also located in Italy and China with some exceptions for some productions of little importance in Europe.

Considering the sheet production waste and eyeglasses production scraps together, we can estimate the European volume equal to about 2,500 tons/year, while in China mention can be made of about 14,000 tons/year.

Not using this waste and these scraps means resorting to the synthesis of new volumes of polymer (based on cellulose and acetic anhydride) and plasticizer necessary for processing, by means of extrusion and solvent, the cellulose acetate plates. This results in a consumption of new polymers for about 16,500 tons/year of polymer and 15,000 tons/ year of cellulose acetate and 6,200 tons/year of diethyl phthalate.

Currently, the only way to recover the waste and scraps from the sector is to gasify the waste, with the production of primary elements such as carbon monoxide, hydrogen, carbon dioxide and water.

From that, the need and value of a process such as that which is the object of the present invention, which is an alternative to the traditional gasification process which includes decomposing the polymers to return to the basic components: carbon monoxide, dioxide, water and hydrogen.

The process of the invention, the pyrolysis, instead allows obtaining intermediates, thus avoiding splitting to carbon oxides and then synthesizing acetic acid again: this advantageously has the direct availability of acetic acid, together with that of ethyl phthalate, which is used in the preparation of cellulose acetate from the polymer, with an evident energy saving.

## BACKGROUND ART

Cellulose acetate (CA) is an environmentally friendly product from production to degradation processes. As all other cellulose derivatives, the synthesis thereof is based on the replacement of the cellulose hydroxyl groups with other functional groups that in this case are the acetyl group.

Although three processes exist for producing cellulose acetate (the process with acetic anhydride, the process with methylene chloride and the heterogeneous process), today only the acetic anhydride process is used, which uses cellulose as a raw material, nature's most abundant renewable source.

The use thereof dates back to the 1930s with studies on synthetic-artificial plastic materials, what we commonly call "plastic" today. It was discovered then that by reacting cellulose not with nitric acid, but with acetic anhydride, a new material is obtained: cellulose acetate. It is non-flammable and the production process is simple and fast. It can perfectly replace the use of celluloid, so that today it is still among the most used materials.

The features of cellulose acetate depend on the average degree of substitution (DS) or on the amount of acetic acid in terms of percentage "acetic acid %". The conversion of DS may be calculated according to the following equation:

$$DS = \frac{162 \ x \ \% \ Acetyl}{(43 \ x \ 100) - [ \ (43 - 1) \ x \ \% \ Acetyl]}$$

where:

-    162 is the molar weight of the cellulose monomer;
-    43 is the molar weight of the acetyl group;
-    % Acetyl is the acetyl content (wt.%).

The relationship between cellulose acetate DS and acetyl content is also reported in the following Table 1.

TABLE 1

Relationship between cellulose acetate DS and combined acetyl and acetic acid content

| Compound | DS | Acetyl Content | Content Acetic acid |
|---|---|---|---|
| Cellulose | | 0.0% | |
| Cellulose Monoacetate | 1 | 21.1% | 29.4% |
| Cellulose Diacetate (CDA) | 0 2 | 34.9% | 48.8% |
| Cellulose Triacetate (CTA) | 3 | 44.8% | 62.5% |

For commercial applications, the cellulose acetate DS is always greater than 2 and typically 2.45. Cellulose acetate needs to be plasticized to be used as a polymer so as to lower the melting temperature thereof, which in the absence of the plasticizer is too close to the decomposition temperature thereof. The use of the plasticizer makes the polymer more flexible, durable and workable, lowering the transition temperature of the macromolecule.

Traditional cellulose acetate plasticizers are triacetin (TA) and diethyl phthalate (DEP). Diethyl phthalate is one of the most common plasticizers of cellulose acetate but, due to some toxicity/safety issues, its industrial use will be reduced in the near future and replaced by the more ecological triacetin plasticizers. A significant amount of plasticizer is generally used (between 25 and 30% by weight depending on the type of use).

The task which the inventors of the present application set themselves was to experimentally evaluate the possibility of chemically recycling cellulose acetate so as to recover valuable compounds such as acetic acid (intermediate for the production of acetic anhydride used in the synthesis of diacetate (DA), cellulose (also in degraded form) and diethyl phthalate that is used as plasticizer.

## CELLULOSE ACETATE DEACETYLATION EXPERIMENTS

Following preliminary research, the task of the inventors was to identify a technology for decomposing cellulose acetate, such as pyrolysis, which had the advantage of producing mainly acetic acid and diethyl phthalate, since the purpose is the recovery of acetic acid, focusing on pyrolysis.

Pyrolysis is a traditional thermochemical process which occurs at medium-high temperatures in an inert atmosphere. Specifically, cellulose acetate decomposes by thermal cracking: at lower temperatures (350-400 °C) acetic acid is released, then cellulose begins to decompose, forming more acetic acid.

PYROLYSIS TREATMENT

Pyrolysis tests were performed in a benchtop fixed bed reactor. Nitrogen was used as a transport gas with a flow rate of 40 mL/min. The exiting gases and vapors were quenched and passed into various traps containing water to condense the vapor phase and absorb the acetic acid in water. The aqueous solution containing acetic acid was analyzed by means of GC-MS and acid-base titrator. The oily phase consisting mainly of diethyl phthalate was analyzed by means of GC-MS. The gas phase was also analyzed by means of a mass spectrometer with different reaction times. Three reaction temperatures (370 °C, 400 °C and 450 °C) were tested.

The following description will be better understood with reference to the accompanying drawings which show, merely by way of non-limiting example, the experimental results obtained.

In the drawings:

fig. 1 is the chromatographic analysis showing the main peak detected, for R.T. of 7.72 acetic acid and for R.T. of 18.43 diethyl phthalate; the other two visible peaks refer to light gaseous compounds.

Figure 2 is the diagram of the entire cellulose acetate process chain comprising chemical recycling.

**EXPERIMENTAL RESULTS**

PYROLYSIS OF CELLULOSE ACETATE

**Pyrolysis product**

To understand the behavior of cellulose acetate under pyrolysis conditions, some preliminary tests were conducted at different temperatures (350 to 500 °C) and 20 min total reaction time. During this period, three gas samples were collected after 5, 10 and 15 minutes and analyzed by means of GC-MS. The main compounds detected were acetic acid and diethyl phthalate. Together with the progress of the reaction, the peak area of the acetic acid decreased, indicating that the acetic acid was released at a low temperature and completely removed in little time.

According to a peculiar feature of the invention, only acetic acid and diethyl phthalate are produced with the pyrolysis process, which is a fundamental advantage considering that the purpose is the recovery of acetic acid. Furthermore, the decomposition of cellulose occurs at higher temperatures, thus the regulation of the temperature and reaction time can be an effective option to make it possible to recover only the two products, avoiding the contamination thereof with the other products originating from the decomposition of cellulose.

The relative amount (% area) of diethyl phthalate and acetic acid as a function of reaction time is shown in Table 1a.

Table 1 a

| Relative amount of pyrolysis products at different reaction times (T=370 °c). | | | |
|---|---|---|---|
| | 5 min | 10 min | 15 min |
| Acetic acid | 13% | 28% | 46% |
| Diethyl phthalate | 87% | 72% | 54% |

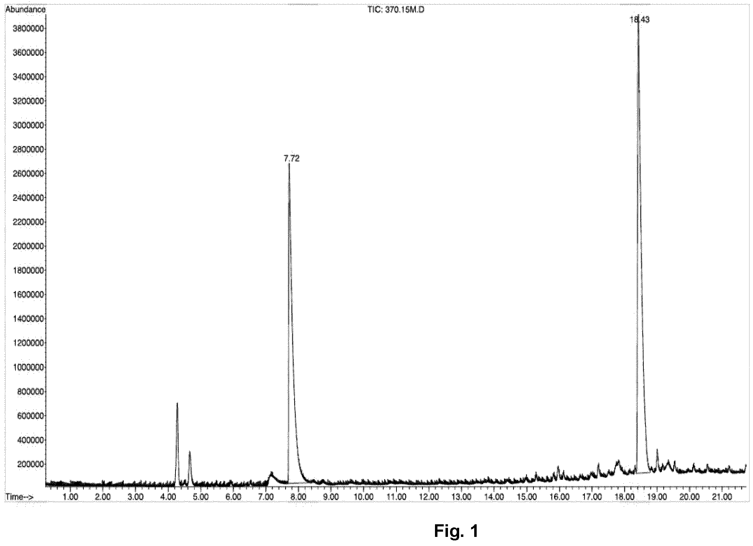

Figure 1 shows the chromatogram of the analysis of the compounds. It is clear that the only visible products are acetic acid and diethyl phthalate.

Based on these preliminary results, a more in-depth trial was conducted and a better recovery and quantification of the product was implemented.

Tests were conducted at three different temperatures (370 °C, 400 °C, 450 °C) to understand the influence of this parameter on cellulose acetate decomposition and acetic acid yield. The tests were conducted by sending all vapor products from the decomposition of cellulose acetate into a cold water trap where most of the acetic acid is solubilized. To avoid product leakage, the residual vapor gases are forced to pass through a second trap with propanol. In the first siphon, the acetic acid in the water is quantified by titration through the measurement of the acidity of water. The products absorbed in the traps were also analyzed by means of GC-MS to investigate in which other compounds they are recovered in addition to acetic acid.

Table 2 shows the acetic acid yields as a function of temperature.

*Table 2: Results in terms of product yield:*

|  | 370 | 400 | 450 |
|---|---|---|---|
| Absolute Acetic Acid (%wt) | 33.53 | 27.03 | 28.91 |
| Relative Acetic Acid (%wt) | 41.92 | 34.31 | 36.14 |
| Solid residue (%wt) | 14.5 | 8.4 | 7.8 |

The data presented in the first and second row of the table are calculated with the following formulas:

1) In the first row, the amount of recovered acetic acid (absolute acetic acid) with respect to the treated raw material was calculated:

$$Acetic\ Acid \quad \frac{Amount\ of\ Acetic\ Acid\ in\ water\ (g)}{Amount\ of\ raw\ material\ (g)} \quad * 100$$

In the second row (relative acetic acid) the amount of acetic acid was measured considering that the cellulose acetate polymer possesses 20% in plasticizer weight (diethyl phthalate) and thus the real cellulose acetate raw material is only 80% of the total:

$$Acetic\ Acid\ \ 2 = \frac{Amount\ of\ Acetic\ Acid\ in\ water\ (g)}{Amount\ of\ raw\ material\ (g) * 0.8} \quad * 100$$

The results reported in Table 2 indicate that the amount of acetic acid decreases with the increase in reaction temperature, in fact, it is reported that after 400 °C, acetic acid undergoes partial decomposition.
The amount of acetic acid recovered considering the raw material extracted from the plasticizer is about 42% at 370°C. This value is perfectly in accordance with the amount of acetic acid in the fresh polymer, which is about 50% of the weight thereof.
The results obtained confirm that with pyrolysis at medium temperature (T<400 °C) it is possible to recover almost all the acetic acid from cellulose acetate. Acetic acid and diethyl phthalate are the two main compounds produced in the tests at 370 °C. Advantageously, the two compounds have very different boiling temperatures and could therefore be easily separated by distillation.
For tests at a higher temperature T>400 °C, an oily phase is also obtained, originating from the decomposition of cellulose. The oily phase represents about 15% of the raw material and mainly consists of diethyl phthalate and compounds from the decomposition of cellulose such as 5-acetoxymethyl-2-furaldehyde, acetyl triethyl citrate, acetic acid, 3,5-diacetoxy-2-ac. and Tri-acetyl-d-mannosan.

Table 3: List of oil compounds with GC-MS analysis.

| Compounds (% area) | T=400 ° C | T=450 °C |
|---|---|---|
| Acetic acid | 20.5 | 10.2 |
| Triacetin | 2.1 | 2.7 |
| 5-Acetoxymethyl-2-furaldehyde | 2.3 | 4.7 |
| Diethyl phthalate | 74.4 | 75.2 |
| Acetyl triethyl citrate |  | 3.7 |
| Acetic Acid 3,5-diacetoxy-2-ac. |  | 1.5 |
| Tri-acetyl-d-mannosan |  | 2.0 |

CONCLUSIONS

From the experimentation conducted on cellulose acetate, it can be concluded that the pyrolysis process can be used for chemically recycling acetic acid not only thanks to the high percentage of acetic acid recovery, but also for the fact that it is the simplest and most effective method to recover both the acetic acid and diethyl phthalate present in the charge.

Under the conditions adopted, the amount of recovered products was greater than 330 g/kg of total feed for acetic acid and about 200 g/kg for diethyl phthalate at the temperature of 370 °C. If the reaction temperatures are increased, cellulose begins to decompose, producing compounds that can contaminate acetic acid and diethyl phthalate, making the separation thereof more difficult. The separation of acetic acid and diethyl phthalate can be easily carried out in a distillation column. The two compounds have very different boiling temperatures (acetic acid 118 °C and diethyl phthalate 302 °C) and did not form any azeotropes, making the separation thereof possible.

Advantageously, the cellulose acetate pyrolysis process can be inserted within a concept of circular economy. Currently, cellulose acetate waste is no longer usable in the process and represents a waste stream. Pyrolysis instead makes this waste stream a source of reagents for the production of new cellulose acetate. The chemical recycling of cellulose acetate to produce diethyl phthalate and acetic acid has made it possible to significantly reduce the environmental impact of the entire production process, decreasing the impact on the production of acetic acid and diethyl phthalate and reusing a waste stream.

Therefore, the object of the present invention is also a closed-cycle plant for producing cellulose acetate eyeglasses frames starting from acetic acid and diethyl phthalate, which includes the recovery thereof by pyrolysis from production waste and the use of the carbonaceous solid residue obtained as a fuel to at least partially support the pyrolysis itself.

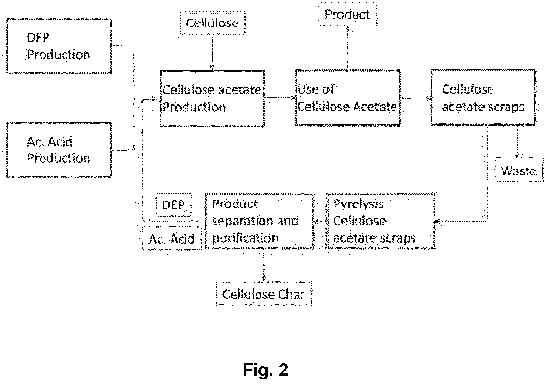

The proposed diagram of the process according to the present invention is shown in Figure 2. The steps of the traditional process are represented with a thin line, the new solution which involves the chemical recycling of cellulose acetate waste is shown in bold. In the latter case, the lower amount of new diethyl phthalate and acetic acid allows lowering the carbon emissions of the entire plant. Furthermore, in addition to the two desired products, the pyrolysis produces a solid residue consisting mainly of deacetylated cellulose. The amount of this solid residue is about 140 g/kg of cellulose acetate and has a calorific value of about 17 MJ/kg, which can be exploited to partially support the pyrolysis process by means of the combustion thereof.

As can be seen from the diagram in fig. 2, the process includes the following steps:

a) subjecting cellulose acetate waste coming from the section where it is processed to pyrolysis (block P) at a temperature between 370 and 400 °C;
b) sending the product exiting from pyrolysis (block P) and consisting of a mixture of acetic acid and diethyl phthalate to a distillation column (block CD) for the separation thereof;
c) mixing both acetic acid and diethyl phthalate to the entering feed stream for the traditional production of cellulose acetate, (block P), reducing the waste only to a carbonaceous solid residue consisting substantially of deacetylated cellulose which, by virtue of the calorific value thereof of about 17MJ/kg, as already indicated, can be exploited to partially support the pyrolysis process by means of the combustion thereof.

**Claims**

1. A process for recovering acetic acid and other valuable materials from cellulose acetate waste, **characterized in that** it includes the following steps:

a) subjecting the cellulose acetate waste from a production process to pyrolysis at a temperature between 370 and 400°C;
b) sending the product exiting from pyrolysis and consisting of a mixture of acetic acid and diethyl phthalate to a distillation column for separation;
c) mixing the acetic acid and diethyl phthalate exiting from said distillation column with the incoming feed stream for the traditional production of cellulose acetate, while the solid carbonaceous residue consisting of deacetylated cellulose, exiting from the same distillation column, is usable to partly support the pyrolysis process by the combustion thereof, lowering the carbon emission of the entire plant.

2. A process according to claim 1 **characterized in that** at a temperature of 370°C the amount of recovered product is of 30% by weight of the total feed for acetic acid and about 20% by weight for diethyl phthalate.

3. A process according to claim 1 **characterized in that** the solid residue exiting from the pyrolysis, mainly consisting of deacetylated cellulose, is about 140 g/kg of cellulose acetate and has a calorific value of about 17 MJ/kg.

4. A process according to claim 1 **characterized in that** the chemical recycling of cellulose acetate to produce diethyl phthalate and acetic acid decreases the environmental impact of the whole production process, reusing a waste flow.

5. A plant for producing eyeglasses, comprising a section for producing cellulose acetate from a primary feed of acetic acid, cellulose and diethyl phthalate, and a section for manufacturing eyeglasses by numerical control machines from flat sheets of cellulose acetate and then producing cellulose acetate waste, **characterized in that** it further comprises: a pyrolysis section where said cellulose acetate waste is decomposed into a mixture of acetic acid and diethyl phthalate, forming a source of reagents for producing new cellulose acetate, and

> a distillation column where said components are separated and purged to be sent to the cellulose acetate production section, joining the acetic acid and diethyl phthalate streams of the primary feed,
> where
> the pyrolysis section produces, in addition to acetic acid and diethyl phthalate, a solid carbonaceous residue mainly consisting of deacetylated cellulose which is usable to partly support the pyrolysis process by the combustion thereof.

**Patentansprüche**

1. Verfahren zur Gewinnung von Essigsäure und anderen Wertstoffen aus Celluloseacetatabfällen, **dadurch gekennzeichnet, dass** es die folgenden Schritte umfasst:

> a) Unterziehen der Celluloseacetatabfälle aus einem Herstellungsprozess einer Pyrolyse bei einer Temperatur zwischen 370 und 400°C;
> b) Weiterleiten des aus der Pyrolyse austretenden Produktes, das aus einem Gemisch aus Essigsäure und Diethylphthalat besteht, in eine Destillationskolonne zur Trennung;
> c) Mischen der Essigsäure und des Diethylphthalats, die aus der Destillationskolonne austreten, mit dem Zufuhrstrom für die herkömmliche Herstellung von Celluloseacetat, während der feste kohlenstoffhaltige Rest, der aus deacetylierter Cellulose besteht und aus derselben Destillationskolonne austritt, zur teilweisen Unterstützung des Pyrolyseprozesses durch dessen Verbrennung verwendet werden kann, wodurch die Kohlenstoffemission der gesamten Anlage verringert wird.

2. Verfahren nach Anspruch 1, **dadurch gekennzeichnet, dass** bei einer Temperatur von 370°C die Menge des zurückgewonnenen Produkts 30 Gew.-% der gesamten Zufuhr für Essigsäure und etwa 20 Gew.-% für Diethylphthalat beträgt.

3. Verfahren nach Anspruch 1, **dadurch gekennzeichnet, dass** der aus der Pyrolyse austretende feste Rest, der hauptsächlich aus deacetylierter Cellulose besteht, etwa 140 g/kg Celluloseacetat ist und einen Heizwert von etwa 17 MJ/kg aufweist.

4. Verfahren nach Anspruch 1, **dadurch gekennzeichnet, dass** das chemische Recycling von Celluloseacetat zur Herstellung von Diethylphthalat und Essigsäure die Umweltauswirkungen des gesamten Herstellungsprozesses verringert und einen Abfallstrom wiederverwendet.

5. Anlage zur Herstellung von Brillen, die einen Abschnitt zur Herstellung von Celluloseacetat aus einer primären Zufuhr von Essigsäure, Cellulose und Diethylphthalat und einen Abschnitt zur Herstellung von Brillen durch numerisch gesteuerte Maschinen aus flachen Blättern von Celluloseacetat und zur anschließenden Herstellung von Celluloseacetatabfall umfasst, **dadurch gekennzeichnet, dass** sie ferner umfasst:

> einen Pyrolyseabschnitt, in dem die Celluloseacetatabfälle in ein Gemisch aus Essigsäure und Diethylphthalat zersetzt werden, das eine Quelle für Reagenzien zur Herstellung von neuem Celluloseacetat bildet, und
> eine Destillationskolonne, in der die genannten Komponenten abgetrennt und gereinigt werden, um sie zusammen mit den Essigsäure- und Diethylphthalatströmen der primären Zufuhr in die Celluloseacetatherstellung weiterzuleiten,
> wobei
> der Pyrolyseabschnitt neben Essigsäure und Diethylphthalat einen festen kohlenstoffhaltiger Rest herstellt, der hauptsächlich aus deacetylierter Zellulose besteht, die zur teilweisen Unterstützung des Pyrolyseprozesses durch deren Verbrennung verwendet werden kann.

**Revendications**

1. Procédé de récupération d'acide acétique et d'autres substances de valeur à partir de déchets d'acétate de cellulose, **caractérisé en ce qu'**il comprend les étapes suivantes :

   a) la soumission des déchets d'acétate de cellulose issus d'un procédé de production à une pyrolyse à une température entre 370 et 400 °C ;
   b) l'envoi du produit sortant de la pyrolyse et consistant en un mélange d'acide acétique et de phtalate de diéthyle vers une colonne de distillation à des fins de séparation ;
   c) le mélange de l'acide acétique et du phtalate de diéthyle sortant de ladite colonne de distillation avec le flux d'alimentation entrant pour la production traditionnelle d'acétate de cellulose, tandis que le résidu carboné solide constitué de cellulose désacétylée, sortant de la même colonne de distillation, est utilisable pour contribuer partiellement au procédé de pyrolyse par sa combustion, pour ainsi réduire les émissions de carbone de l'ensemble de l'installation.

2. Procédé selon la revendication 1, **caractérisé en ce qu'**à une température de 370 °C, la quantité de produit récupéré est de 30 % en poids de la charge d'alimentation totale pour l'acide acétique et d'environ 20 % en poids pour le phtalate de diéthyle.

3. Procédé selon la revendication 1, **caractérisé en ce que** le résidu solide sortant de la pyrolyse, principalement constitué de cellulose désacétylée, est d'environ 140 g/kg d'acétate de cellulose et présente une valeur calorifique d'environ 17 MJ/kg.

4. Procédé selon la revendication 1, **caractérisé en ce que** le recyclage chimique de l'acétate de cellulose pour produire du phtalate de diéthyle et de l'acide acétique diminue l'impact environnemental de l'ensemble du procédé de production, par la réutilisation d'un flux de déchets.

5. Installation de fabrication de lunettes, comprenant une section de production d'acétate de cellulose à partir d'une charge d'alimentation primaire d'acide acétique, de cellulose et de phtalate de diéthyle, et une section de fabrication de lunettes par machines à commande numérique à partir de feuilles plates d'acétate de cellulose, puis de production de déchets d'acétate de cellulose, **caractérisée en ce qu'**elle comprend en outre : une section de pyrolyse dans laquelle lesdits déchets d'acétate de cellulose sont décomposés en un mélange d'acide acétique et de phtalate de diéthyle, formant une source de réactifs pour la production d'un nouvel acétate de cellulose, et

   une colonne de distillation dans laquelle lesdits composants sont séparés et purgés pour être envoyés à la section de production d'acétate de cellulose, rejoignant les flux d'acide acétique et de phtalate de diéthyle de la charge d'alimentation primaire,
   où
   la section de pyrolyse produit, en plus de l'acide acétique et du phtalate de diéthyle, un résidu carboné solide constitué principalement de cellulose désacétylée qui est utilisable pour contribuer partiellement au procédé de pyrolyse par sa combustion.

Fig. 1

**Fig. 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 112047827 A